# EUROPEAN PATENT SPECIFICATION

(11) **EP 1 838 262 B1**
(45) Date of publication and mention of the grant of the patent: **02.08.2017**
(21) Application number: 05818615.6
(22) Date of filing: 07.12.2005
(51) Int. Cl.: A61F 9/04, A61K 9/00, A61K 36/55, A61K 131/00, A61P 27/02

(54) **LINSEED EYE COMPRESSES**
LEINSAMEN-AUGENKOMPRESSEN
COMPRESSES OCULAIRES AUX GRAINES DE LIN

(30) Priority: 08.12.2004 GB 0426850
(43) Date of publication of application: 03.10.2007
(73) Proprietor: James, Teifion Emlyn, Halifax, West Yorkshire, HX3 0BB (GB)
(72) Inventor: JAMES, Teifion Emlyn, West Yorkshire HX3 0BB (GB); GRANT, Susan, West Yorkshire HD8 0NB (GB)
(74) Representative: Appleyard Lees IP LLP
(86) International application number: PCT/GB2005/004676
(87) International publication number: WO 2006/061596

(56) References cited:
- WO-A-99/32047
- HUMPHREY W T: "Flaxseeds in ophthalmic folk medicine." AMERICAN JOURNAL OF OPHTHALMOLOGY. AUG 1970, vol. 70, no. 2, August 1970 (1970-08), pages 287-290, XP009062832 ISSN: 0002-9394

## Description

This invention relates to a medical device and particularly, although not exclusively, relates to a device for treatment of common eye conditions, such as Meibomian Gland Dysfunction (MGD), Meibomian Gland Disease, Meibomian Gland Inspissation, Stye, Hordeolum, Chalazion, MGD associated tear film instability, consequent dry eye, Blepharitis and Blepharoconjunctivitis.

The meibomian glands secrete lipid into the tears and the lipid forms the oily layer of the precorneal tear film which reduces tear evaporation. Several eye conditions are attributable to incorrect functioning of the meibomian glands. For example, obstructive-type MGD is characterised by inspissation of meibomian gland lipid, resulting in hyposecretion of lipid into tears. MGD is known to be a major cause of ocular surface abnormalities and ocular discomfort.

Other eye conditions, such as blepharitis, may be caused by bacterial infection or a generalised skin condition. Symptoms of the disease include eye irritation, burning, tearing, foreign body sensation, crusty debris (in the lashes in the corner of the eyes or on the lids), dryness and red eyelid margins.

Numerous different treatments for common eye conditions have been proposed. For example, Eye Contact Lens 29(2): 96-9, 2003 Apr, discloses the use of warm, moist compress therapy for the treatment of MGD. Br.J. Ophthalmol 86(12):1403-7, 2002 Dec discloses the use of an infrared warm compression device in the treatment of MGD. Ann Ophthalmol 20(5):196-8, 1998 May, discloses the effectiveness of intralesional steroid insection in the treatment of chalazia. Int. Ophthalmol Clin 24(2):65-77, 1984, Summer, discloses the use of warm compresses, eyelid scrubs and topical antibiotics in the treatment of blepharoconjunctivitis. Trans Am Acad Ophthalmol Otolaryngol 83(5):866, 1997 Sept-Oct, discloses a battery-operated warm compress for chalazion therapy.

Whilst numerous proposals have been made for treating eye conditions, there is still a need for a simple, cheap, re-useable device which can easily be used by patients in their own homes, with minimal risk of mis-use.

WO99/32047 discloses eye pillows with an adjustable strap for treatment of accu-pressure points around a user's eyes.

HUMPHREY W T, "Flaxseeds in ophthalmic folk medicine.", AMERICAN JOURNAL OF OPHTHALMOLOGY. AUG 1970, (197008), vol. 70, no. 2, ISSN 0002-9394, pages 287 - 290, discloses use of flax seed meal dressings for the treatment of chronic or acute abscesses.

It is an object of the present invention to provide a medical device for inter alia treatment of common eye conditions.

According to a first aspect of the invention, there is provided a medical device as described in claim 1. Suitably, at least 80wt%, preferably at least 90wt%, more preferably at least 95wt%, especially at least 99wt% of material contained within the receptacle comprises said seeds.

Preferably, said receptacle includes less than 5wt%, more preferably less than 1wt%, especially substantially no material in addition to said seeds which is a lachrymator substance - i.e. a substance which causes tear production. Preferably, said receptacle does not contain any material in addition to said seeds which produces, consists of or contains a volatile oil. For the avoidance of doubt, said seeds contained in said receptacle will include oil(s) as natural constituents thereof and such oil(s) preferably are present in said seeds contained in said receptacle. As described hereafter, in use, the device is heated. Preferably, scented oils (or the like) are not produced on heating the device from materials other than said seeds, since such oils could be detrimental to the treatment of eye conditions by virtue of them being pro-irritants.

At least 20%, suitably at least 30%, preferably at least 40%, more preferably at least 45%, especially at least about 50% of the volume of the receptacle is filled with said seeds.

The seeds suitably fill less than 80%, preferably less than 70%, more preferably less than 60%, especially less than 55% of the volume of the receptacle.

Preferably, the seeds occupy 40-60% of the volume of the receptacle. This allows the seeds to be redistributed easily within the receptacle after heating to equalise heat throughout the mass of seeds and thereby reduce the risk of the device having "hot spots" which could be hazardous to its safe use.

Said seeds are preferably not crushed and/or are substantially whole. The aforementioned volumes therefore suitably refer to the volumes occupied by such uncrushed/whole seeds.

The volume of seeds in the receptacle is preferably at least 200ml, preferably at least 250ml. The volume may be less than 400ml, preferably less than 350ml, more preferably less than 300ml.

The weight of seeds in the receptacle may be at least 100g, preferably at least 125g. The weight may be less than 250g, preferably less than 200g, more preferably less than 175g.

The seeds are preferably dried seeds.

The density of the seeds in the receptacle is preferably in the range 0.4 to 0.8 ml/g, more preferably in the range 0.5 to 0.7 ml/g.

Said receptacle preferably includes a first face which is arranged to overlie, conform to and contact a person's closed eyes and nose in use. Said first face preferably comprises a first material which is relatively lightweight. Suitably, greater than 50% by area, preferably greater than 75% by area, more preferably greater than 90% by area of said first face is made up of said first material. Preferably said first face is made up substantially entirely of said first material.

Said first material may have a density of less than 1.5 g/l, preferably less than 1.3 g/l. The density may be at least 1.1 g/l.

Said first material may comprise synthetic or naturally occurring fibres. Preferably, it includes naturally occurring fibres. More preferably it comprises silk fibres.

Said first material suitably comprises silk and more preferably comprises substantially pure silk. Silk is preferred since it facilitates conformance of the device to the shape of a patient's face. Furthermore, heat can readily be conducted through it, in use, from seeds within the receptacle to a patient using the device.

Said first face may have an area of at least 100cm², preferably at least 150cm². The area may be less than 300cm², preferably less than 250cm², especially about 200cm².

Said receptacle suitably includes a second face which is arranged to face away from a patient's eyes/nose in use. Thus, said second face preferably faces in an opposite direction compared to said first face.

Said second face preferably comprises a second material which is preferably different to said first material suitably by being of a different weight or being made of a different type of fabric. Said second material may be of heavier weight compared to said first material. The difference in the respective weights (or densities) of the first and second materials may help to achieve a satisfactory contact pressure of the first face of the device on a patient's eyes and the surrounding areas and, additionally, helps the first face of the device to conform to the shape of a patient's face.

Suitably, greater than 50% by area, preferably greater than 75% by area, more preferably greater than 90% by area of said second face is made up of said second material. Preferably, said second face is made up substantially entirely of said second material.

Suitably, greater than 50% by area, preferably greater than 75% by area, more preferably greater than 90% by area of said second face is black. This may reduce radiation of heat outwardly from said second face and facilitate loss of heat from the device via said first face, to a patient in use.

In a preferred embodiment, the first face is made of a material which dissipates heat, for example to a patient's eyes in use, at a greater rate than that at which said second face dissipates heat outwardly away from the device.

Preferably, the ratio of the weight of material which makes up said second face to the weight of material which makes up said first face is at least 1.1, preferably at least 1.5.

Said device (preferably the first face thereof) is suitably arranged to overlie and contact the eyes and nose of a patient's face in use. The device suitably includes a central region which is arranged to overlie the patient's nose and first and second regions on opposite sides of said central region which are arranged to overlie a patient's eyes (although, for the avoidance of doubt there need not be a clear dividing line between said central and first and second regions). The maximum width of the central region (which width extends in a direction down a patient's face in use) is preferably greater than the maximum width of said first and second regions. The shape may facilitate contact of the device with the patient's eyes/peri-orbital tissues and/or help the device (especially the first face thereof) conform to and/or apply heat in use to appropriate areas.

The maximum width of said central region may be at least 6cm, preferably at least 7cm, more preferably at least 8cm, especially at least 9cm. The maximum width may be less than 15cm, preferably less than 14cm, more preferably less than 12cm.

The central region preferably does not define an isthmus; it preferably defines the maximum width of the device.

The central region preferably includes a first convex outwardly facing part and preferably includes a second convex outwardly facing part, wherein the first and second convex parts suitably facing in substantially opposite directions.

The first region preferably includes a first convex outwardly facing part and preferably includes a second convex outwardly facing part, wherein the first and second convex parts suitably face in substantially opposite directions.

The second region preferably includes a first convex outwardly facing part and preferably includes a second convex outwardly facing part, wherein the first and second convex parts suitably face in substantially opposite directions.

Preferably, the first convex parts of the central region, and first and second regions together define a first continuous convex outwardly facing part of the device; and preferably, the second convex parts of the central region and first and second regions together define a second continuous outwardly facing part of the device.

Preferably, said first and second continuous convex outwardly facing parts face generally in substantially opposite directions but curve towards one another and meet at respective outer edges, preferably to define apical or pointed regions of the device.

Preferably, the device is elliptical in plan view.

Said device suitably has a maximum length of at least 18cm, preferably at least 20cm, more preferably at least 22cm, especially at least 24cm. The length may be less than 35cm, preferably less than 30cm, more preferably less than 28cm. The length of the device is suitably such that, in use, edges of the device extend around the sides of a patient's face. This arrangement may improve the contact of the device with and the transfer of heat to the eyes/peri-orbital tissues.

The total weight of the device may be at least 100g, suitably at least 125g, preferably at least 150g, more preferably at least 165g. The weight may be less than 250g, preferably less than 225g, more preferably less than 200g, especially less than 185g.

The device described may advantageously be used to treat eye conditions such as Meibomian Gland Dysfunction (MGD), Meibomian Gland Disease, Meibomian Gland Inspissation, Stye, Hordeolum, Chalazion, MGD associated tear film instability, consequent dry eye, Blepharitis and Blepharoconjunctivitis.

According to another aspect, there is provided the use according to claim 13.

The uses described may involve heating the device.

According to an embodiment described herein, there is provided a method of treatment of Meibomian Gland Dysfunction (MGD), the method comprises:
(i) selecting a medical device according to said first aspect;
(ii) causing the seeds of said device to become heated; and
(iii) contacting a surface of the device with a patient's face so that it overlies the patient's eyes and nose, whereby heat is transferred from the device to the patient.

In step (ii), the seeds are preferably heated by microwave energy, for example using a domestic microwave oven.

Preferably prior to step (iii), the device is manipulated to cause the seeds to flow within the receptacle and redistribute heat between the seeds.

According to an embodiment described herein, there is provided a pack comprising a medical device according to the first aspect. Suitably, the device is provided in a receptacle. The pack may include instructions for use of the medical device.

Specific embodiments of the invention will now be described, by way of example, with reference to the accompanying schematic drawings, in which:

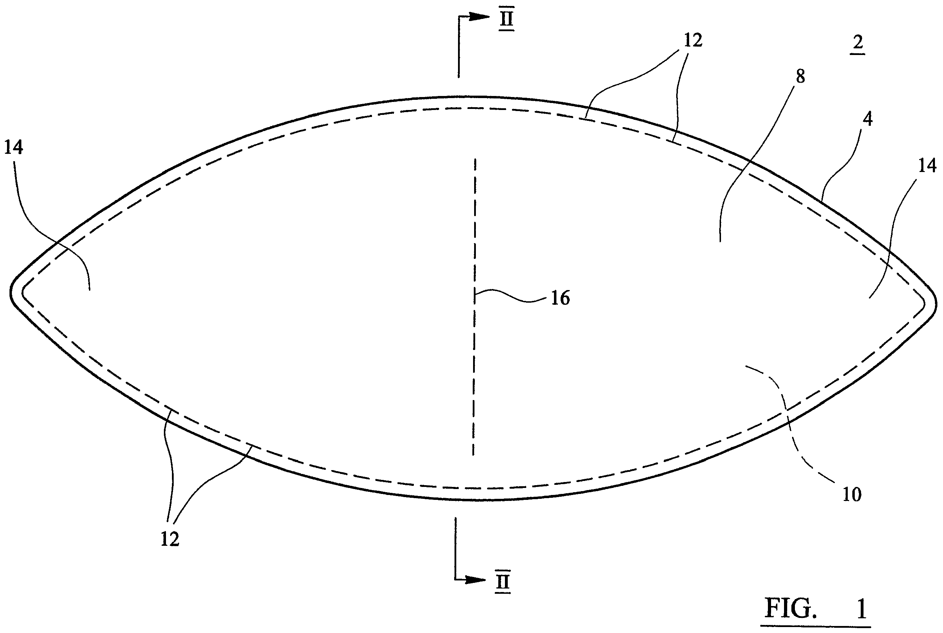

Figure 1 is a plan view of a medical device for treating common eye conditions;
Figure 2 is a cross-section about line II-II of Figure 1; and
Figure 3 shows the medical device in use.

The medical device 2 comprises an elliptical sack 4 which contains flax seeds 6. The device may be microwaved in a domestic microwave oven to heat the flax seeds. Thereafter, the device may be placed on a patient's face over his/her eyes to treat several common eye conditions by delivery of heat to the peri-orbital tissues, principally the skin of the eyelids and the glands therein.

The device 2 is made from two ellipse-shaped pieces of material 8, 10. Piece 8 is made of silver-coloured silk and piece 10 is made of a heavier material such as black suedex. The two pieces 8, 10 are sewn together around their peripheries to define a part-closed sack which is then filled with flax seeds 6. The sack may then be oversewn around its edge (see stitches 12) to seal the flax seeds 6 therewithin.

Suitably, the maximum width of the pieces of material, as measured between opposing lines of stitching is in the range 8-16cm, preferably 12 to 14cm, and the maximum length of the pieces of material as measured between opposition lines of stitching is in the range 20 to 35cm, preferably about 25 to 30cm.

The sack 4 is filled to about 50% of its capacity with flax seeds 6.

The flax seeds may be of any origin and suitably comprise dry seeds.

The sack 4 preferably only contains flax seeds and does not contain any additional material which may include components such as essential oils, which would volatilise on heating and may be detrimental to treatment of eye conditions, and indeed provoke ocular irritation, discomfort, allergy or sensitivity.

In use, the device 2 is placed in a domestic microwave oven and microwaved at about 800W for 20 to 30 seconds. On removal from the oven, the device is manipulated to cause the flax seeds to move from side to side thereby ensuring substantially even distribution of heat within the device and eliminating potentially hazardous hot spots. The shape of device 2 facilitates quick and efficient redistribution of the flax seeds and, since the device does not include any constriction or isthmus, the seeds do not become trapped within any area of the device. Furthermore, the device can easily be manipulated so the seeds are substantially evenly distributed throughout it prior to application to a patient's face.

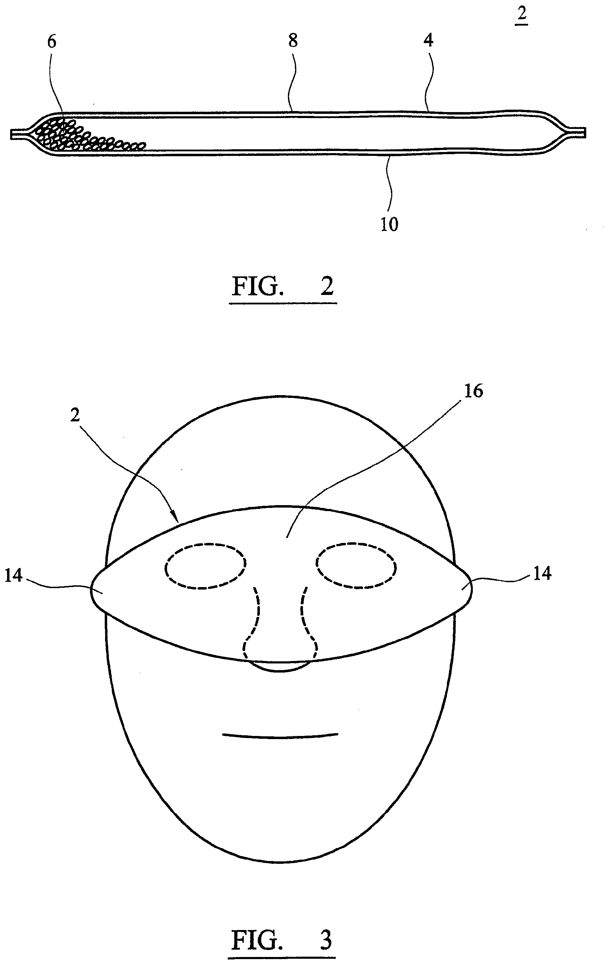

With the device having attained an appropriate temperature and with a patient lying down or reclining, or seated with the neck extended, the device is placed on his/her face, with silk piece 8 contacting the face, so that a central region adjacent imaginary line 16 of the device overlies part of the patient's forehead and nose; and outer regions 14 extend 2 to 6cm around respective sides of the face, as represented in Figure 3. As a consequence of the density of the device and the fineness of the silk piece 8, the device (more particularly the silk piece thereof) conforms well to the shape of the face so that it makes direct contact with the patient's eyelids and surrounding areas, including the nose and cheek bones. Heat is then readily conducted from the flax seeds to the patient's eyelids and surrounding areas thereby to treat a relevant eye condition.

Advantageously, it is found that the shape of the device is such that it is able to conform to and be used effectively for a wide range of patients of greatly different facial anatomies.

Advantageously, the device can be re-used many times, over a period of several months.

A non-randomized, open-label, qualitative study of 30 consecutive patients with significant Meibomian Gland Dysfunction has been undertaken to assess efficacy of the device. Under test conditions 30 patients were enrolled in a qualitative study to determine whether MGD sufferers received symptomatic relief by using the device. The patients enrolled in the study used the device 2 daily.

Patients used the device for a minimum of ten minutes each day and consecutively for a minimum of seven days. Of the 30 patients enrolled in the study, 3 patients derived no subjective benefit from the treatment. Of the 27 patients who noticed a subjective improvement in their symptoms, 24 had noted a marked improvement in their symptoms (graded as highly significant) whereas the remaining 3 patients noted a mild to moderate improvement in their symptoms.

It was concluded that 10% of patients using the device 2 appear to derive no subjective benefit. A further 10% derived mild to moderate symptomatic relief. 80% of patients derived significant or highly significant subjective symptomatic relief.

One female patient derived exceptional benefit from the use of the device 2. On cessation of treatment her symptoms recurred. On re-instigating treatment with the device the symptoms again resolved. This experience has been mirrored in several other instances. In reviewing patients from the initial cohort, patients have learned to titrate the use of the device against their symptoms.

## Claims

1. A medical device for use in treatment of Meibomian Gland Dysfunction (MGD), the device comprising a flexible receptacle containing seeds of the plant Linum usitatissimum, for example flax seeds.

2. A medical device for use according to claim 1, wherein said flexible receptacle is arranged to overlie and contact the eyes and nose of a patient in use and includes a central region arranged to overlie a patient's nose and first and second regions on opposite sides of the central region which are arranged to overlie a patient's eyes, wherein the maximum width of the central region is greater than the maximum width of the first and second regions, wherein seeds can be moved from side to side within the receptacle and wherein the receptacle does not include any isthmus which may restrict redistribution of seeds within the receptacle.

3. A medical device for use according to claim 1 or claim 2, wherein said receptacle includes substantially no material in addition to said seeds which is a lachrymator substance

4. A medical device for use according to any preceding claim, wherein at least 80wt% of material contained within the receptacle comprises said seeds; wherein the seeds occupy 40-60% of the volume of the receptacle; wherein the volume of seeds in the receptacle is at least 200ml and is less than 400ml; wherein the weight of seeds in the receptacle is at least 100g and is less than 250g; and wherein the density of the seeds in the receptacle is in the range 0.4 to 0.8 ml/g.

5. A medical device for use according to any preceding claim, wherein said receptacle includes a first face which is arranged to overlie, conform to and contact a person's closed eyes and nose in use, wherein said first face comprises a first material which has a density of less than 1.5 g/l; wherein said first face has an area of at least 100cm² and less than 300cm²; and wherein the first face is made of a material which dissipates heat at a greater rate than that at which said second face dissipates heat.

6. A medical device for use according to any claim dependent on claim 2, wherein the maximum width of said central region is at least 6cm and is less than 15cm.

7. A medical device for use according to any claim dependent on claim 2, wherein said central region includes a first convex outwardly facing part and a second convex outwardly facing part, wherein said first and second convex parts face in substantially opposite directions; said first region includes a first convex outwardly facing part and a second convex outwardly facing part, wherein said first and second convex parts face in substantially opposite directions; and said second region includes a first convex outwardly facing part and a second convex outwardly facing part, wherein said first and second convex parts face in substantially opposite directions.

8. A medical device for use according to claim 7, wherein said respective first convex outwardly facing parts of the central region, first region and second region together define a first continuous convex outwardly facing part of the device.

9. A medical device for use according to claim 7 or claim 8, wherein the second convex outwardly facing parts of the central region, first region and second region together define a second continuous convex outwardly facing part of the device.

10. A medical device for use according to claim 9, wherein said first and second continuous convex outwardly facing parts face generally in substantially opposite directions but curve towards one another and meet at respective outer edges.

11. A medical device for use according to any preceding claim, which is elliptical in plan view.

12. A medical device for use according to any preceding claim, wherein the device has a maximum length of at least 18cm and less than 35cm; and wherein the total weight of the device is at least 100g and is less than 250g.

13. The use of a flexible receptacle and seeds of the plant Linum usitatissimum for the manufacture of a medical device for treatment of Meibomian Gland Dysfunction (MGD).

## Patentansprüche

1. Medizinische Vorrichtung für die Verwendung bei der Behandlung von Meibom-Drüsen-Dysfunktion (MGD), wobei die Vorrichtung einen flexiblen Behälter umfasst, der Samen der Pflanze Linum usitatissimum enthält, beispielsweise Flachssamen.

2. Medizinische Vorrichtung für die Verwendung gemäß Anspruch 1, wobei der flexible Behälter dafür gestaltet ist, in Verwendung die Augen und die Nase eines Patienten zu bedecken und in Kontakt damit zu sein, und einen zentralen Bereich umfasst, der dafür gestaltet ist, die Nase eines Patienten zu bedecken, und erste und zweite Bereiche an gegenüberliegenden Seiten des zentralen Bereichs, die dafür gestaltet sind, die Augen eines Patienten zu bedecken, wobei die größte Breite des zentralen Bereichs größer als die größte Breite des ersten und des zweiten Bereichs ist, wobei die Samen innerhalb des Behälters von einer Seite auf die andere bewegt werden können und der Behälter keine Engstelle enthält, die die Umverteilung von Samen innerhalb des Behälters beschränken kann.

3. Medizinische Vorrichtung für die Verwendung gemäß Anspruch 1 oder Anspruch 2, wobei der Behälter im Wesentlichen kein Material zusätzlich zu den Samen enthält, das ein Lachrymatorstoff ist.

4. Medizinische Vorrichtung für die Verwendung gemäß einem der vorstehenden Ansprüche, wobei wenigstens 80 Gew.-% des in dem Behälter enthaltenen Materials die Samen umfasst; wobei die Samen 40-60 % des Volumens des Behälters besetzen; wobei das Volumen der Samen in dem Behälter wenigstens 200 ml beträgt und weniger als 400 ml beträgt; wobei das Gewicht der Samen in dem Behälter wenigstens 100 g beträgt und weniger als 250 g beträgt; und wobei die Dichte der Samen in dem Behälter in dem Bereich von 0,4 bis 0,8 ml/g liegt.

5. Medizinische Vorrichtung für die Verwendung gemäß einem der vorstehenden Ansprüche, wobei der Behälter eine erste Seite umfasst, die dafür gestaltet ist, in Verwendung die geschlossenen Augen und die Nase einer Person zu bedecken, an sie angepasst und in Kontakt damit zu sein, wobei die erste Seite ein erstes Material umfasst, das eine Dichte von weniger als 1,5 g/l aufweist; wobei die erste Seite eine Fläche von wenigstens 100 cm² und weniger als 300 cm² aufweist; und wobei die erste Seite aus einem Material besteht, das Wärme mit einer größeren Rate ableitet als die zweite Seite Wärme ableitet.

6. Medizinische Vorrichtung für die Verwendung gemäß einem der Ansprüche abhängig von Anspruch 2, wobei die größte Breite des zentralen Bereichs wenigstens 6 cm beträgt und kleiner als 15 cm ist.

7. Medizinische Vorrichtung für die Verwendung gemäß einem der Ansprüche abhängig von Anspruch 2, wobei der zentrale Bereich einen ersten konvexen, nach außen zeigenden Teil und einen zweiten konvexen, nach außen zeigenden Teil aufweist, wobei der erste und der zweite konvexe Teil in im Wesentlichen entgegengesetzte Richtungen zeigen; wobei der erste Bereich einen ersten konvexen, nach außen zeigenden Teil und einen zweiten konvexen, nach außen zeigenden Teil aufweist, wobei der erste und der zweite konvexe Teil in im Wesentlichen entgegengesetzte Richtungen zeigen; und der zweite Bereich einen ersten konvexen, nach außen zeigenden Teil und einen zweiten konvexen, nach außen zeigenden Teil aufweist, wobei der erste und der zweite konvexe Teil in im Wesentlichen entgegengesetzte Richtungen zeigen.

8. Medizinische Vorrichtung für die Verwendung gemäß Anspruch 7, wobei die jeweiligen ersten konvexen, nach außen zeigenden Teile des zentralen Bereichs, des ersten Bereichs und des zweiten Bereichs zusammen einen ersten durchgehenden konvexen, nach außen zeigenden Teil der Vorrichtung definieren.

9. Medizinische Vorrichtung für die Verwendung gemäß Anspruch 7 oder Anspruch 8, wobei die zweiten konvexen, nach außen zeigenden Teile des zentralen Bereichs, des ersten Bereichs und des zweiten Bereichs zusammen einen zweiten durchgehenden konvexen, nach außen zeigenden Teil der Vorrichtung definieren.

10. Medizinische Vorrichtung für die Verwendung gemäß Anspruch 9, wobei der erste und der zweite durchgehende konvexe, nach außen zeigende Teil in im Wesentlichen entgegengesetzte Richtungen zeigen, aber sich zueinander krümmen und sich an entsprechenden Außenrändern treffen.

11. Medizinische Vorrichtung für die Verwendung gemäß einem der vorstehenden Ansprüche, die in Draufsicht elliptisch ist.

12. Medizinische Vorrichtung für die Verwendung gemäß einem der vorstehenden Ansprüche, wobei die Vorrichtung eine größte Länge von wenigstens 18 cm und weniger als 35 cm aufweist; und wobei das Gesamtgewicht der Vorrichtung wenigstens 100 g beträgt und kleiner als 250 g ist.

13. Verwendung eines flexiblen Behälters und von Samen der Pflanze Linum usitatissimum zum Herstellen einer medizinischen Vorrichtung für die Behandlung von Meibom-Drüsen-Dysfunktion (MGD).

## Revendications

1. Dispositif médical destiné à une utilisation dans le traitement d'un dysfonctionnement des glandes de Meibomius (DGM), le dispositif comprenant un réceptacle souple contenant des graines de la plante *Linum usitatissimum,* par exemple des graines de lin.

2. Dispositif médical destiné à une utilisation selon la revendication 1, dans lequel ledit réceptacle souple est conçu pour recouvrir et venir en contact avec les yeux et le nez d'un patient lors de l'utilisation et comprend une région centrale conçue pour recouvrir le nez d'un patient et des première et seconde régions sur des côtés opposés de la région centrale qui sont conçues pour recouvrir les yeux d'un patient, dans lequel la largeur maximale de la région centrale est supérieure à la largeur maximale des première et seconde régions, dans lequel les graines peuvent être déplacées d'un côté à l'autre à l'intérieur du réceptacle et dans lequel le réceptacle n'inclut pas d'isthme qui pourrait limiter la redistribution des graines à l'intérieur du réceptacle.

3. Dispositif médical destiné à une utilisation selon la revendication 1 ou la revendication 2, dans lequel ledit réceptacle ne comprend essentiellement aucun autre matériau outre lesdites graines qui soit une substance lacrymogène.

4. Dispositif médical destiné à une utilisation selon l'une quelconque des revendications précédentes, dans lequel au moins 80 % en poids du matériau présent à l'intérieur du réceptacle comprend lesdites graines ; dans lequel les graines occupent 40 à 60 % du volume du réceptacle ; dans lequel le volume des graines dans le réceptacle est d'au moins 200 ml et est inférieur à 400 ml ; dans lequel le poids des graines dans le réceptacle est d'au moins 100 g et est inférieur à 250 g ; et dans lequel la densité des graines dans le réceptacle se situe dans la plage de 0,4 à 0,8 ml/g.

5. Dispositif médical destiné à une utilisation selon l'une quelconque des revendications précédentes, dans lequel ledit réceptacle comprend une première face qui est conçue pour, lors de l'utilisation, recouvrir les yeux fermés et le nez d'une personne, s'adapter à leur forme et venir en contact avec ceux-ci, dans lequel ladite première face comprend un premier matériau qui présente une densité inférieure à 1,5 g/l ; dans lequel ladite première face présente une surface d'au moins 100 cm² et inférieure à 300 cm² ; et dans lequel la première face est constituée d'un matériau qui dissipe la chaleur à une vitesse supérieure à celle à laquelle ladite seconde face dissipe la chaleur.

6. Dispositif médical destiné à une utilisation selon l'une quelconque des revendications dépendant de la revendication 2, dans lequel la largeur maximale de ladite région centrale est d'au moins 6 cm et est inférieure à 15 cm.

7. Dispositif médical destiné à une utilisation selon l'une quelconque des revendications dépendant de la revendication 2, dans lequel ladite région centrale comprend une première partie convexe orientée vers l'extérieur et une seconde partie convexe orientée vers l'extérieur, lesdites première et seconde parties convexes étant orientées dans des directions essentiellement opposées ; ladite première région comprend une première partie convexe orientée vers l'extérieur et une seconde partie convexe orientée vers l'extérieur, lesdites première et seconde parties convexes étant orientées dans des directions essentiellement opposées ; et ladite seconde région comprend une première partie convexe orientée vers l'extérieur et une seconde partie convexe orientée vers l'extérieur, lesdites première et seconde parties convexes étant orientées dans des directions essentiellement opposées.

8. Dispositif médical destiné à une utilisation selon la revendication 7, dans lequel lesdites premières parties convexes orientées vers l'extérieur respectives de la région centrale, de la première région et de la seconde région définissent conjointement une première partie convexe orientée vers l'extérieur continue du dispositif.

9. Dispositif médical destiné à une utilisation selon la revendication 7 ou la revendication 8, dans lequel les secondes parties convexes orientées vers l'extérieur de la région centrale, de la première région et de la seconde région définissent conjointement une seconde partie convexe orientée vers l'extérieur continue du dispositif.

10. Dispositif médical destiné à une utilisation selon la revendication 9, dans lequel lesdites première et seconde parties convexes orientées vers l'extérieur continues sont globalement orientées dans des directions essentiellement opposées mais s'incurvent l'une en direction de l'autre et se rejoignent au niveau de bords extérieurs respectifs.

11. Dispositif médical destiné à une utilisation selon l'une quelconque des revendications précédentes, qui est elliptique en vue en plan.

12. Dispositif médical destiné à une utilisation selon l'une quelconque des revendications précédentes, le dispositif présentant une longueur maximale d'au moins 18 cm et inférieure à 35 cm ; et le poids total du dispositif étant d'au moins 100 g et inférieur à 250 g.

13. Utilisation d'un réceptacle souple et de graines de la plante *Linum usitatissimum* pour la fabrication d'un dispositif médical pour le traitement d'un dysfonctionnement des glandes de Meibomius (DGM).
